# FASCICULE DE BREVET EUROPEEN

(11) **EP 3 313 477 B1**
(45) Date de publication et mention de la délivrance du brevet: **31.07.2019**
(21) Numéro de dépôt: 16731147.1
(22) Date de dépôt: 22.06.2016
(51) Int. Cl.: A61M 5/178, A61M 5/32

(54) **SERINGUE À AIGUILLE COLLÉE**
SPRITZE MIT EINER GEBUNDENEN NADEL
SYRINGE COMPRISING A BONDED NEEDLE

(30) Priorité: 23.06.2015 FR 1555748
(43) Date de publication de la demande: 02.05.2018
(73) Titulaire: Biocorp Production, 63500 Issoire (FR)
(72) Inventeur: ANEAS, Antoine, 63200 Menetrol (FR)
(74) Mandataire: Lavoix
(86) Numéro de dépôt international: PCT/EP2016/064374
(87) Numéro de publication internationale: WO 2016/207196

(56) Documents cités:
- WO-A1-2009/137845
- WO-A1-2013/134465
- US-A1- 2009 299 295

## Description

La présente invention concerne une seringue à aiguille collée, notamment une seringue du type pré-remplie.

WO-A-2013/134 465 divulgue une seringue à aiguille collée équipée d'un protège-aiguille. Ce protège-aiguille est un embout souple dans lequel est enfoncée l'aiguille. Il a pour fonction de conserver l'aiguille propre, c'est-à-dire d'éviter la contamination d'un principe actif renfermé dans le corps de seringue et de protéger l'aiguille contre toute agression mécanique extérieure. La seringue comprend également un système de sécurité après usage, qui a pour fonction de protéger l'aiguille en fin d'injection. Cela permet d'éviter de se blesser avec l'aiguille lorsque celle-ci est retirée du corps du patient et de lutter contre la transmission de maladies, comme le VIH.

Le système de sécurité comprend un manchon extérieur qui est mobile selon un axe longitudinal de la seringue à l'encontre d'un effort élastique généré par un ressort, entre une position avancée, ou il recouvre l'aiguille, et une position reculée, où l'aiguille est découverte. Le système de sécurité comprend également un collier, qui est monté libre en rotation autour d'une partie d'extrémité du corps de seringue. Ce collier comporte un pion qui est engagé dans une ouverture radiale du manchon, laquelle forme un guide pour le pion. Lors d'une injection, le manchon est en appui contre la peau du patient et ne peut alors plus tourner autour de son axe de révolution pour guider le pion dans l'ouverture. Le fait que le collier soit monté libre en rotation autour de la partie d'extrémité du corps de seringue permet donc de guider le pion dans l'ouverture du manchon, bien que ce dernier soit immobile en rotation autour de son axe.

Avec le matériel décrit dans WO-A-2013/134 465, un utilisateur mal informé peut faire tourner le manchon extérieur autour de son axe, en entrainant ainsi le collier en rotation autour de la partie d'extrémité du corps de seringue. L'embout souple est fixé sur le collier, de sorte qu'il tourne conjointement avec le collier. Des copeaux de matière sont donc susceptibles de pénétrer à l'intérieur de l'aiguille et de contaminer le principe actif lors de l'injection car l'aiguille creuse comporte une extrémité taillée en biseau.

En outre, un jeu radial est défini entre le collier et la partie d'extrémité du corps de seringue pour permettre la rotation du collier. Il existe donc aussi un jeu radial entre l'embout souple et la partie d'extrémité du corps de seringue. Des bactéries peuvent donc s'infiltrer à travers ce jeu radial dans le volume interne de l'embout, c'est-à-dire à proximité de l'aiguille. Ces bactéries peuvent alors pénétrer dans l'aiguille lorsque l'embout est retiré.

C'est à ces inconvénients qu'entend plus particulièrement remédier l'invention en proposant une seringue à aiguille collée avec laquelle les risques de contamination du principe actif sont moindres.

A cet effet, l'invention concerne une seringue à aiguille collée, comprenant un corps de seringue, un protège-aiguille, comprenant un embout souple, un manchon extérieur, qui est mobile le long d'un axe longitudinal entre une position avancée, où il recouvre l'aiguille, et une position reculée, où il ne recouvre pas l'aiguille, et un collier, qui est monté avec un jeu radial autour d'une partie d'extrémité du corps de seringue et qui comporte au moins un pion engagé dans une ouverture de guidage du manchon. Conformément à l'invention, une portion de l'embout souple remplit le jeu radial entre la partie d'extrémité du corps de seringue et le collier lorsque l'embout est monté sur la seringue, de sorte que le collier est immobilisé en rotation autour de la partie d'extrémité du corps de seringue tant que l'embout est monté sur la seringue.

Grâce à l'invention, l'embout est disposé de telle sorte qu'il y a suffisamment d'adhérence pour empêcher le collier de tourner autour de cette dernière lorsqu'il est monté sur l'aiguille. Ainsi, le manchon extérieur du système de sécurité est également bloqué en rotation autour de son axe tant que l'embout souple n'a pas été retiré de l'aiguille, ce qui limite les risques de contamination.

Selon des aspects avantageux mais non obligatoires de l'invention, une seringue à aiguille collée peut comprendre une ou plusieurs des caractéristiques suivantes, prises dans toute combinaison techniquement admissible :
- La portion de l'embout souple est comprimée radialement entre la partie d'extrémité du corps de seringue et le collier lorsque le protège-aiguille est monté sur la seringue ;
- Le protège aiguille comprend une gaine rigide enveloppant l'embout souple ;
- La gaine est en deux parties détachables l'une de l'autre par un mouvement de rotation relatif entre les deux parties ou par un mouvement axial relatif entre les deux parties, alors que le collier forme une première partie de la gaine ;
- Un jeu radial existe entre l'embout souple et la deuxième partie de la gaine ;
- L'embout et la gaine sont liés en translation parallèlement à l'axe longitudinal ;
- La gaine comprend deux épaulements annulaires opposés qui délimitent entre eux un volume de réception d'un bourrelet annulaire appartenant à l'embout souple ;
- Le collier comprend des pattes d'accrochage sur le corps de seringue, ces pattes ne s'opposant pas à la rotation du collier autour de la partie d'extrémité du corps de seringue lorsque l'embout est retiré de l'aiguille ;
- La seringue comprend des moyens de verrouillage du manchon en position avancée en fin d'injection.
- La seringue est une seringue pré-remplie.

L'invention et d'autres avantages de celle-ci apparaitront plus clairement à la lumière de la description qui va suivre d'un mode de réalisation d'une seringue à aiguille collée conforme à son principe, faite uniquement à titre d'exemple et en référence aux dessins annexés dans lesquels :
- les figures 1, 3, 5, 7, 9, 11 et 13 représentent chacune une vue de côté d'une seringue à aiguille collée conforme à l'invention, lors d'une étape particulière d'injection,
- les figures 2, 4, 6, 8, 10, 12 et 14 représentent chacune une coupe longitudinale de la seringue correspondant aux figures mentionnées ci-dessus,
- la figure 15 est une coupe partielle longitudinale et à plus grande échelle selon la ligne XV-XV à la figure 2,
- les figures 16 à 18 sont des coupes longitudinales représentant des étapes d'assemblage du dispositif de la figure 15 sur une seringue à aiguille collée.

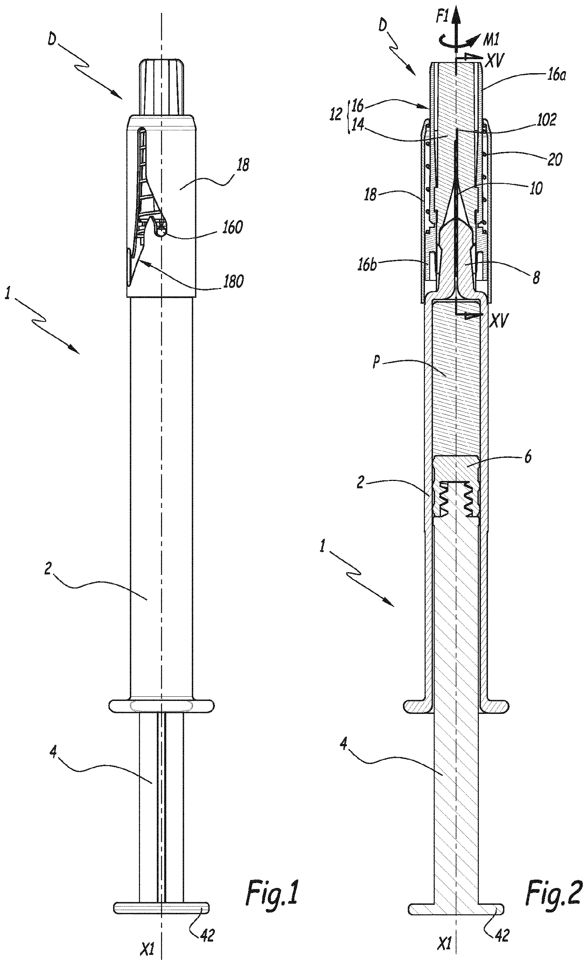

Sur chacune des figures 1 à 14 est représentée une seringue à aiguille collée 1. Cette seringue 1 est du type pré-remplie et s'étend selon un axe longitudinal X1. Elle comprend un corps de seringue 2 en verre, qui est globalement tubulaire et centré sur l'axe X1. Le corps 2 comporte nez de seringue 8 ayant un décrochement externe 80. Le nez de seringue 8 comprend une partie d'extrémité distale 82 qui s'étend à partir du décrochement externe 80.

Une aiguille creuse 10 est collée à l'intérieur d'un trou axial traversant le nez 8, c'est à dire débouchant dans le volume interne du corps de seringue 2, dans lequel est stocké un principe actif P, tel qu'un médicament. L'aiguille 10 comporte une extrémité distale 102 taillée en biseau.

La seringue 1 comporte également une tige de piston 4 qui est liée en translation avec un joint d'étanchéité 6. Plus précisément, la tige 4 comporte une extrémité filetée qui est vissée à l'intérieur d'un taraudage prévu dans le joint 6. Le joint 6 sert de piston pour éjecter le principe actif P à travers l'aiguille creuse 10, c'est pourquoi le joint 6 est couramment appelé « piston » ou « joint de piston ». A l'extrémité axiale opposée du joint 6, la tige 4 comprend une palette 42 sur laquelle l'utilisateur peut exercer un effort de poussée vers le nez 8. La tige 4 est déplaçable en translation par rapport au corps 2 selon l'axe X1, c'est-à-dire qu'elle est apte à coulisser à l'intérieur du corps de seringue 2.

Dans cette description, la direction avant ou distale désigne une direction parallèle à l'axe longitudinal X1 et tournée vers l'épiderme du patient dans des conditions normales d'utilisation de la seringue 1, alors que la direction arrière ou proximale est orientée à l'opposé par rapport à la zone d'injection, du côté de la palette 42.

La seringue 1 comporte, à l'avant, un dispositif D de protection de l'aiguille 10. Ce dispositif D est adapté pour être monté sur le nez 8 de la seringue 2. Le dispositif D comprend un protège-aiguille 12 qui permet, d'une part, de conserver l'aiguille 10 propre avant l'utilisation de la seringue 1 et, d'autre part, de protéger l'aiguille 10 contre toute action mécanique extérieure. Par exemple, le protège-aiguille 12 empêche l'aiguille 10 de se tordre ou de se briser avant usage.

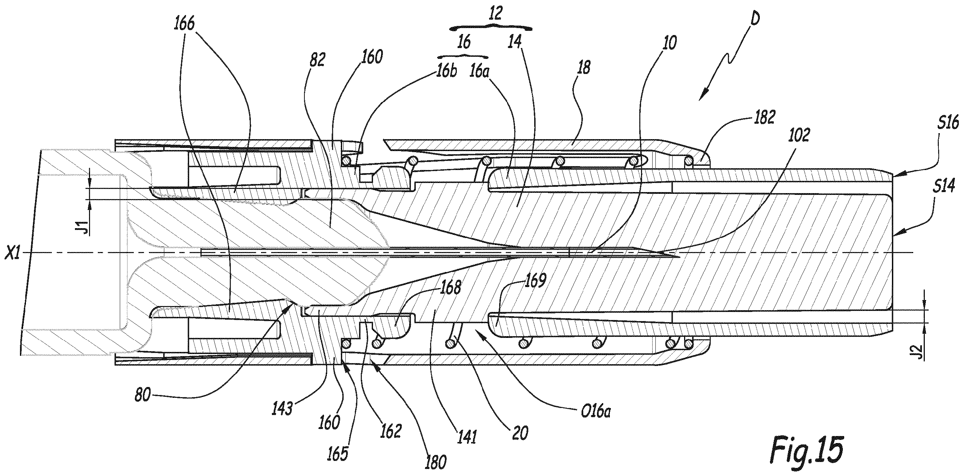

Le protège-aiguille 12 est rigide et comprend un embout souple 14 dans lequel est enfoncée l'aiguille 10 et une gaine rigide 16 qui enveloppe l'embout 14. L'embout 14 est en élastomère, par exemple en caoutchouc ou en matière thermoplastique injectable, alors que la gaine rigide 16 est en matière plastique, dans l'exemple en polyéthylène à haute densité (PEHD). Le protège-aiguille 12 est mieux visible à la figure 15. Comme visible sur cette figure, l'embout 14 comporte un bourrelet annulaire 141 et, à l'arrière de de bourrelet, une jupe 143 entourant la partie d'extrémité 82 du nez 8.

La gaine rigide 16 est en deux parties 16a et 16b qui sont détachables l'une de l'autre par un mouvement de rotation relatif entre ces deux parties. En effet, les deux parties de la gaine 16a et 16b sont reliées entre elles par des pontets sécables 162, conçus pour être rompus lors de l'application d'un moment M1 de rotation relative entre les deux parties de la gaine 16a et 16b. La partie 16a est disposée à l'avant de la partie 16b. Les parties 16a et 16b sont chacune de forme tubulaire centrée sur l'axe longitudinal X1.

L'embout 14 et la partie avant 16a de la gaine 16 sont liés en translation selon l'axe X1. En effet, le bourrelet 141 fait saillie radialement à l'intérieur d'un logement périphérique 016a ménagé sur la surface radiale interne de la partie 16a. Ce logement périphérique 016a est délimité par deux épaulements 168 et 169 opposés axialement l'un à l'autre. Comme visible à la figure 15, le logement 016a est débouchant vers l'extérieur de la gaine 16, sur une partie de la circonférence de cette gaine. Sur la partie de la gaine visible à la figure 2, le logement 016a ne débouche pas vers l'extérieur de la gaine 16. Le caractère débouchant du logement 016a est facultatif.

L'embout 14 peut être monté par l'avant à l'intérieur de la gaine 16 en comprimant temporairement radialement le bourrelet 141. Il existe un jeu radial J2 entre la surface extérieure de l'embout 14 et la surface intérieure de la partie 16a de la gaine 16. Comme l'embout 14 présente un diamètre qui diminue en allant vers l'avant, le jeu radial J2 correspondant diminue en allant vers l'arrière. L'embout 14 est donc agencé à l'intérieur de la partie 16a de manière qu'il n'est pas solidaire en rotation avec cette dernière. Par ailleurs, les surfaces axiales d'extrémité avant S14 et S16, respectivement de l'embout 14 et de la gaine 16, sont affleurantes.

La partie arrière 16b de la gaine 16 forme un collier qui est encliqueté autour du nez 8 de la seringue 1. Le collier 16b comprend donc des moyens de fixation autour du nez 8 de la seringue 1 formés par des pattes élastiques 166 conformées pour venir se coincer dans un décrochement 80 du nez 8. Ces pattes 166 ne s'opposent pas à la rotation du collier 16b autour de la partie d'extrémité 82 du corps de seringue 2 lorsque l'embout 14 est retiré de l'aiguille. Le collier 16b comporte aussi deux pions 160 diamétralement opposés, qui font saillie radialement vers l'extérieur par rapport à l'axe longitudinal X1. Il existe un jeu radial J1 entre le collier 16b et la partie d'extrémité 82 du nez 8.

Lorsque l'embout 14 est en place sur la seringue 1, la jupe 143 de l'embout 14 remplit le jeu radial J1 entre le collier 16b et la partie d'extrémité 82 du nez 8. Plus précisément, la jupe 143 est comprimée radialement entre la partie d'extrémité 82 et le collier 16b. Cela permet d'assurer l'étanchéité entre le volume interne de l'embout 14 et l'extérieur et d'éviter ainsi l'infiltration de bactéries à proximité de l'aiguille, comme cela pourrait se produire avec le matériel de WO-A-2013/134 465. En outre, l'adhérence entre l'embout 14 et le collier 16b empêche ce dernier de tourner autour de la partie d'extrémité 82 du nez 8. En revanche, dès que l'embout 14 est retiré de la seringue 1, le collier 16b est libre en rotation autour du nez 8 du fait du jeu radial J2 existant entre les deux pièces.

Le dispositif de protection D comporte également un système de sécurité après usage, qui a pour fonction de protéger l'aiguille en fin d'injection. Ce système de sécurité comprend un manchon extérieur 18, qui est disposé coaxialement autour de la gaine rigide 16. Le manchon 18 est fabriqué en matériau opaque, pour cacher complètement l'aiguille 10. Ce manchon 18 délimite un bord radial interne 182 au niveau de son extrémité avant et deux ouvertures 180 dans lesquelles les pions 160 sont respectivement engagés. Dans l'exemple, les pions 160 ne font pas saillie à l'extérieur des ouvertures 160. Les ouvertures 180 servent de guide aux pions 160. Chaque ouverture 180 est globalement en forme de Y asymétrique, avec les branches du Y qui s'étendent vers l'arrière. Les branches du Y sont référencées 180a et 180c, alors que sa portion centrale est référencée 180b. Cette portion centrale 180b est une portion droite, c'est-à-dire un couloir. Le dispositif D comprend également des moyens de verrouillage du manchon 18 en position avancée, qui sont activés en fin d'injection. Dans l'exemple, ces moyens de verrouillage sont formés par un logement 180d qui s'étend, à partir de la branche 180c, vers l'avant.

Le manchon extérieur 18 est mobile axialement, c'est-à-dire le long de l'axe X1, entre une position avancée, où il recouvre l'aiguille 10 et une position reculée, où l'aiguille 10 est découverte. Le système de sécurité comprend des moyens de rappel élastique du manchon extérieur 18 en position avancée. Ces moyens de rappel comprennent un ressort hélicoïdal 20 qui est intercalé entre le rebord axial interne 182 du manchon 18 et un épaulement 165 formé sur la partie arrière 16b de la gaine rigide 16. Le ressort hélicoïdal 20 présente un pas à droite, c'est-à-dire que le sens d'enroulement du ressort 20 est à droite. Cela signifie que le ressort 20 est enroulé à droite, ou dans le sens horaire, lorsque l'on regarde le ressort 20 par le bas aux figures 1 à 14, c'est-à-dire du côté arrière.

Ci-dessous sont décrites différentes étapes d'utilisation de la seringue 1 en référence aux figures 1 à 14.

Tout d'abord, l'utilisateur doit retirer le protège-aiguille rigide 12 pour pouvoir réaliser l'injection. Pour ce faire, il applique le moment M1 autour de l'axe X1, comme représenté à la figure 2, pour faire tourner la partie avant 16a par rapport à la partie arrière 16b et rompre les pontets 162. Ceci est possible car la partie arrière 16b de la gaine 16, c'est-à-dire le collier, est immobile en rotation autour du nez de seringue 8 du fait de l'adhérence avec la jupe 143 de l'embout 14. Une fois les pontets 162 rompus, l'utilisateur peut retirer la partie avant 16a de la gaine rigide 16, comme représenté par la flèche F1 à la figure 2. Le retrait de la partie 16a entraine solidairement le retrait de l'embout 14 par coopération du bourrelet 141 avec l'épaulement arrière 168 du logement 016a.

Le collier 16b peut alors librement tourner autour du nez 8 car le jeu radial J1 n'est plus rempli par la jupe 143 de l'embout 14. L'embout 14 et la partie avant 16a de la gaine 16 sont retirés de la seringue 1 par une translation selon la flèche F1, sans faire tourner l'embout 14 autour de l'aiguille 10, de manière que l'extrémité distale 102 de l'aiguille 10, taillée en biseau, ne forme pas de copeaux de matière susceptibles de pénétrer dans l'aiguille 10.

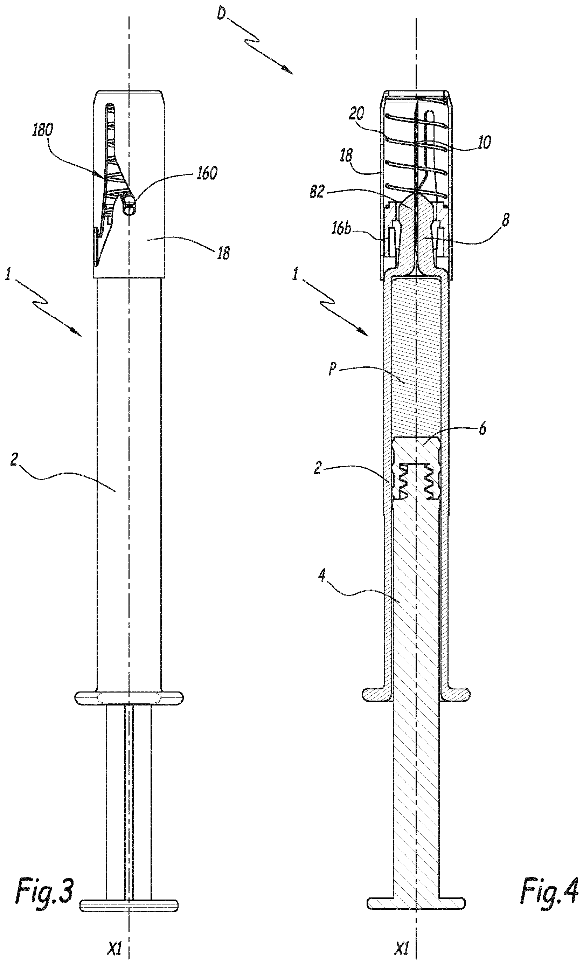

Le retrait de l'embout 14 et de la partie avant 16a de la gaine 16 amène la seringue 1 dans la configuration des figures 3 et 4. Dans cette configuration, l'aiguille 10 est entièrement recouverte par le manchon 18. Tant que la seringue 1 n'a pas été utilisée, les pions 160 de la partie arrière 16b de la gaine 16 sont logés dans la branche 180a des évidements 180.

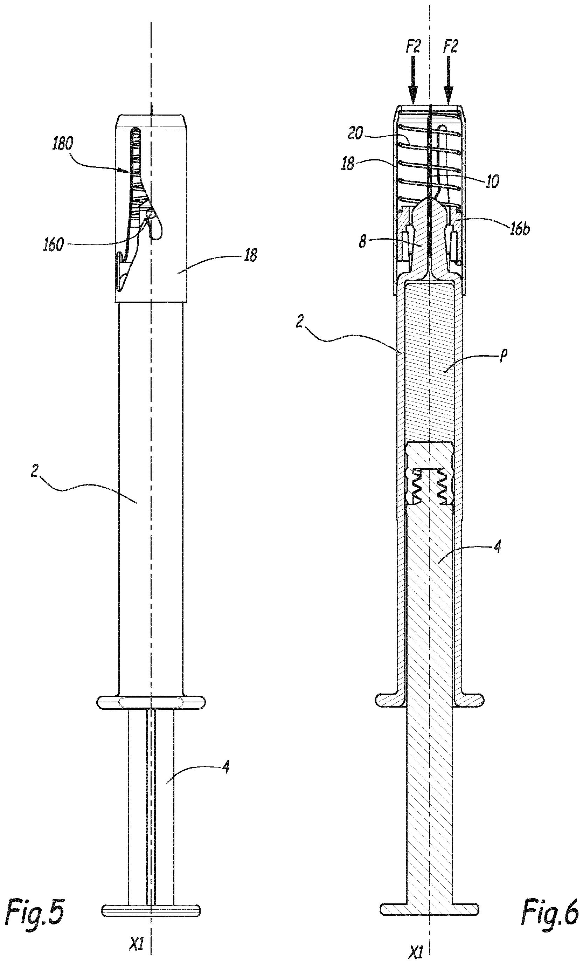

En référence aux figures 5 et 6, lorsque la seringue 1 est amenée contre l'épiderme d'un patient, l'adhérence entre l'extrémité avant du manchon 18 et la peau empêche le manchon 18 de tourner autour de son axe. La pression exercée par le manchon 18 sur la peau entraine le recul du manchon 18, comme représenté par les flèches F2 à la figure 6. Le ressort 20 est alors comprimé, l'aiguille 10 pénètre dans l'épiderme et les pions 160 se déplacent de la branche 180a jusque dans la portion centrale 180b. Le déplacement des pions 160 à l'intérieur des ouvertures 180 est possible car le collier 16b portant les pions 160 est libre de tourner autour du nez 8.

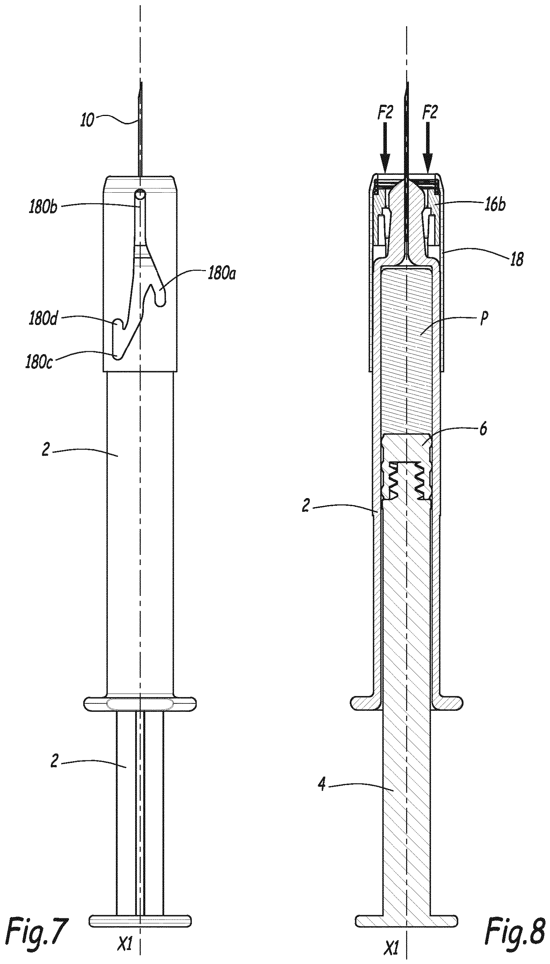

Le manchon 18 recule alors autour du corps de seringue 2 et l'aiguille 10 n'est pas découverte tant que la seringue 1 n'est pas amenée contre l'épiderme du patient, à la différence des matériels selon WO-A-2013/134465 et WO-A-2007/077463, où l'aiguille est en partie découverte avant que la seringue ne soit amenée contre le corps du patient. Autrement dit, le manchon 18 n'est pas reculé préalablement à l'injection pour laisser apparaitre l'aiguille 10. Ainsi, il n'y a pas de risque de piqûre accidentelle avant l'injection. La poursuite du mouvement amène le manchon extérieur 18 vers sa position reculée, dans laquelle il ne recouvre plus l'aiguille 10. La poursuite du mouvement s'effectue jusqu'à ce que les pions 160 arrivent au fond du couloir 180b des évidements 180, comme représenté aux figures 7 et 8.

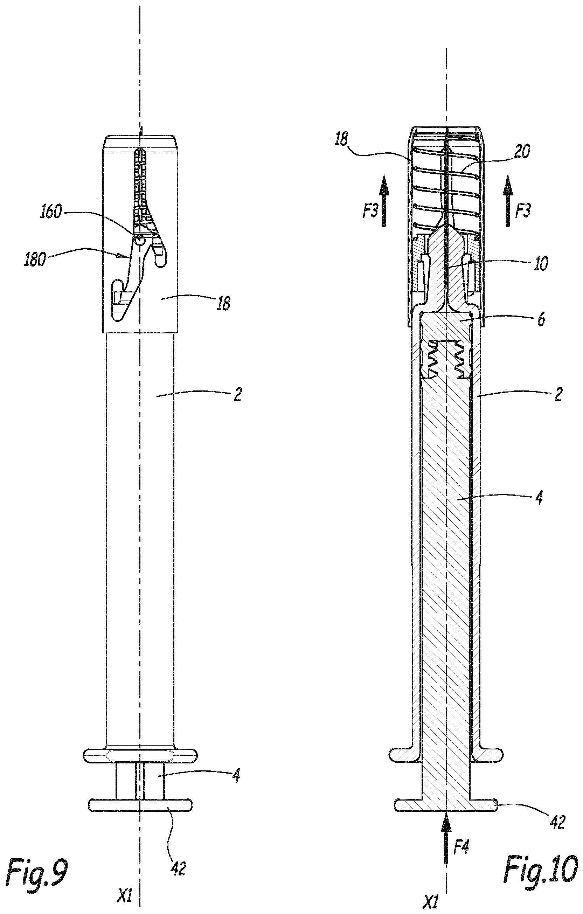

Dans la configuration des figures 7 et 8, l'aiguille 10 de la seringue 1 est complètement enfoncée dans l'épiderme du patient. L'utilisateur peut alors appuyer sur la palette 42 de la tige 4 pour éjecter le principe actif P contenu à l'intérieur de la seringue 1 dans le corps du patient, comme représenté par la flèche F4 à la figure 10.

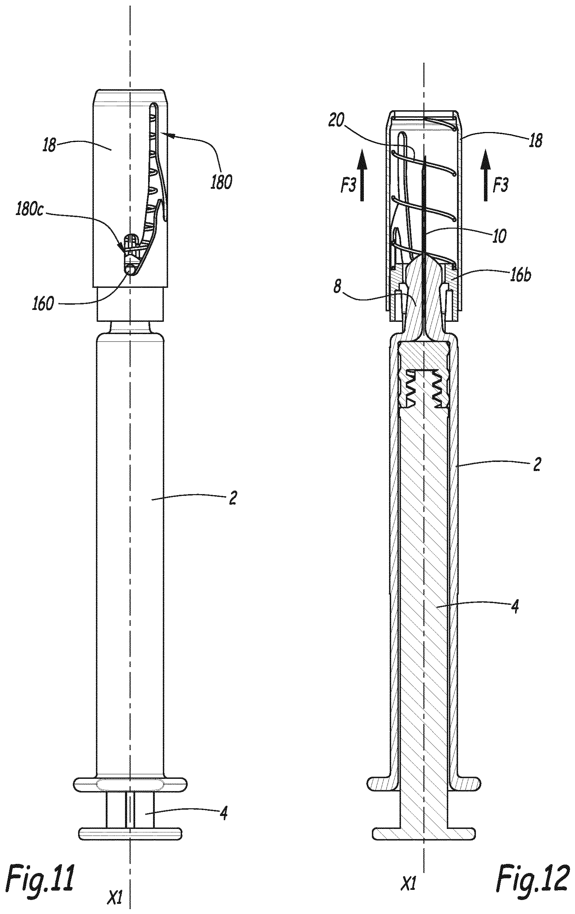

Lorsque l'utilisateur retire la seringue 1 du corps du patient, le manchon extérieur 18 est rappelé élastiquement en position avancée par le ressort 20, comme représenté par les flèches F3 à la figure 10. Le manchon extérieur 18 revient alors en recouvrement de l'aiguille 10 et les pions 160 coulissent dans le couloir 180b des évidements 180 en direction de la branche 180c. La seringue 1 se trouve alors dans la configuration des figures 11 et 12, qui correspond à une configuration de fin d'injection.

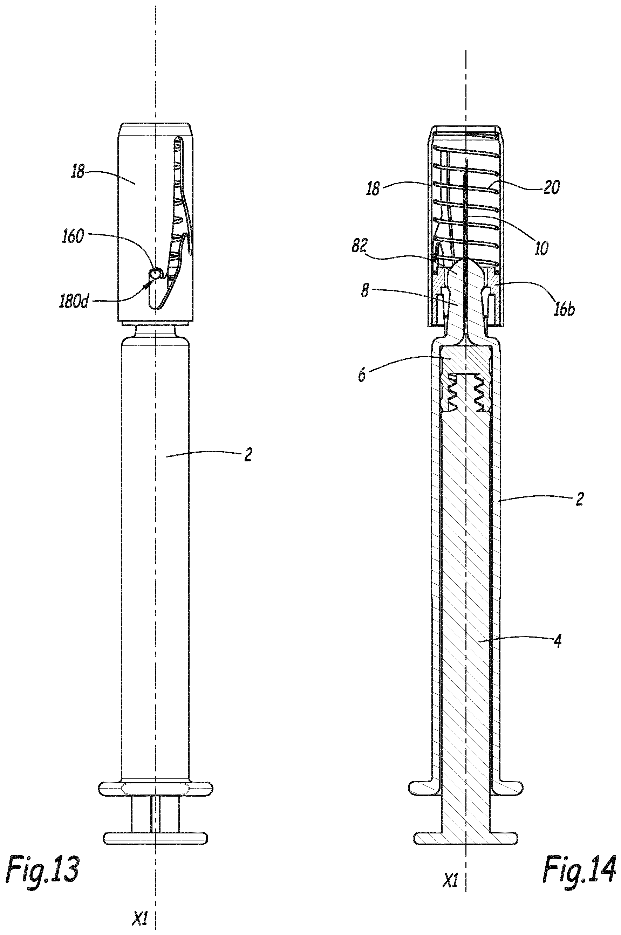

Si, après usage de la seringue 1, un utilisateur maladroit appuie sur le manchon 18, c'est-à-dire tente de reculer le manchon 18, les pions 160 se déplacent alors dans le logement 180d des évidements 180 et le déplacement du manchon 18 vers l'arrière est bloqué, comme représenté sur les figures 13 et 14. Cela constitue une sécurité supplémentaire puisque l'aiguille 10 ne peut plus être découverte en fin d'injection. Plus précisément, le déplacement des pions 160 de la branche 180c vers le logement 180d est favorisé du fait que le ressort 20 a un sens d'enroulement à droite. En effet, ce ressort 20 exerce, lorsqu'il est comprimé, un couple sur le manchon 18 qui est dirigé, du fait de son sens d'enroulement, dans le sens antihoraire en vue de dessus à la figure 11, c'est-à-dire lorsque l'on regarde la seringue 1 du côté de l'aiguille 10. Ce couple permet d'éviter que les pions 160 retournent en direction du couloir 160b si l'utilisateur tente de reculer le manchon 18 après l'injection. Ce couple permet également de guider correctement les pions 160 dans le couloir 180b jusqu'à la branche 180c des évidements 180 puisque le ressort 20 exerce un couple sur le collier 16b qui est dirigé, du fait de son sens d'enroulement, dans le sens horaire en vue de dessus à la figure 9, c'est-à-dire lorsque l'on regarde la seringue 1 du côté de l'aiguille 10.

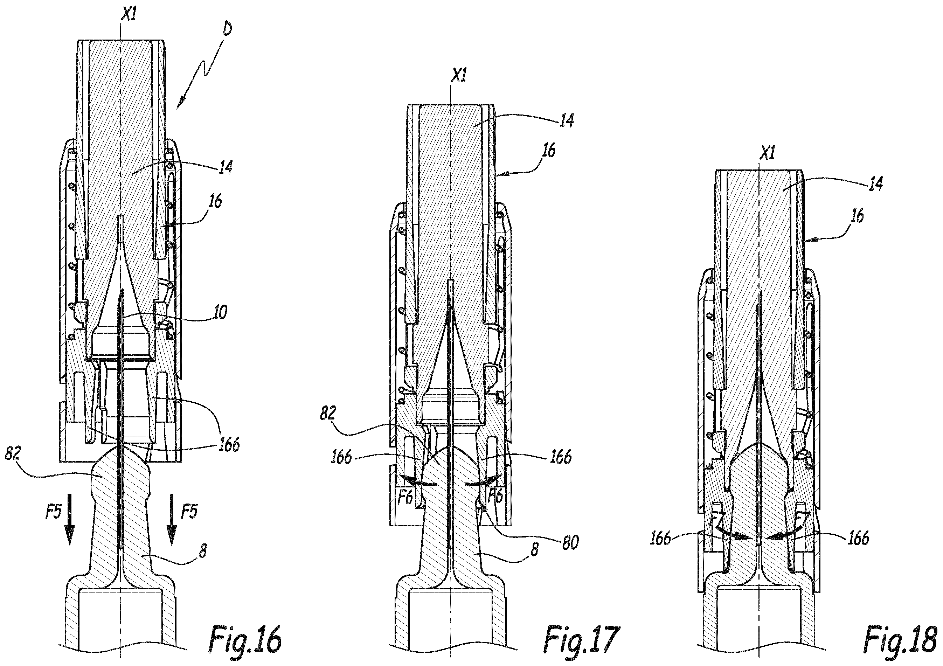

Sur les figures 16 à 18 sont représentées les étapes de montage du dispositif de protection D sur le nez 8 du corps de seringue 2. Une première étape du montage du dispositif de protection D consiste à rapprocher le dispositif D du nez 8, comme représenté par la flèche F5 à la figure 16. En poursuivant le mouvement d'approche dans le sens de la flèche F5, les pattes élastiques 166 sont alors déformées selon une direction radiale centrifuge F6 au contact du nez 8, comme visible à la figure 17. Une fois que les pattes 166 ont dépassé le décrochement 80 du nez 8, elles viennent s'encliqueter contre ce dernier par retour élastique de la matière, comme représenté par les flèches F7 à la figure 18. Le nez 8 de la seringue 1 est conformé pour bloquer le dégagement des pattes 166.

En variante non représentée, d'autres moyens de rappel qu'un ressort peuvent être envisagés pour rappeler le manchon extérieur 18 en position avancée en fin d'injection.

En variante non représentée, un seul évidement 180 est pratiqué dans le manchon 18. De même, le manchon peut délimiter un nombre d'évidements 180 strictement supérieur à deux, par exemple égal à trois.

En variante non représentée, les parties 16a et 16b de la gaine 16 peuvent être vissées l'une à l'autre ou liées par un autre mécanisme de verrouillage rotatif, par exemple du type à baïonnette. Dans tous les cas, les parties 16a et 16b sont détachables l'une de l'autre par un mouvement de rotation relatif entre les deux parties.

En variante, la tige 4 et le joint 6 sont dissociés, c'est-à-dire que la tige 4 est simplement prévue pour pousser le joint 6.

Selon une autre variante non représentée, les deux parties 16a et 16b de la gaine 16 sont détachables l'une de l'autre par un mouvement axial relatif entre les deux parties. Par exemple, la partie avant 16b peut être détachée du collier 16a simplement en tirant la partie 16b du côté opposé au collier 16a ou en faisant basculer la partie 16b par rapport au collier 16a, c'est-à-dire en désaxant la partie 16b par rapport à un axe central du collier 16a.

Les caractéristiques des variantes et modes de réalisation envisagés ci-dessus peuvent être combinées entre elles pour générer de nouveaux modes de réalisation de l'invention dans le cadre des revendications.

## Revendications

1. Seringue (1) à aiguille collée (10), comprenant :
- un corps (2) de seringue,
- un protège-aiguille (12), comprenant un embout souple (14),
- un manchon extérieur (18), qui est mobile le long d'un axe longitudinal (X1) entre une position avancée, où il recouvre l'aiguille, et une position reculée, où il ne recouvre pas l'aiguille, et
- un collier (16b), qui est monté avec un jeu radial (J1) autour d'une partie d'extrémité (82) du corps de seringue (2) et qui comporte au moins un pion (160) engagé dans une ouverture de guidage (180) du manchon,
**caractérisée en ce qu'**une portion (143) de l'embout souple (14) remplit le jeu radial (J1) entre la partie d'extrémité (82) du corps de seringue et le collier (16b) lorsque l'embout (14) est monté sur la seringue, de sorte que le collier est immobilisé en rotation autour de la partie d'extrémité du corps de seringue tant que l'embout (14) est monté sur la seringue.

2. Seringue selon la revendication 1, **caractérisée en ce que** la portion (143) de l'embout souple (14) est comprimée radialement entre la partie d'extrémité (82) du corps de seringue et le collier (16b) lorsque le protège-aiguille (12) est monté sur la seringue.

3. Seringue selon la revendication 1 ou 2, **caractérisée en ce que** le protège aiguille (12) comprend une gaine rigide (16) enveloppant l'embout souple (14).

4. Seringue selon la revendication 3, **caractérisée en ce que** la gaine est en deux parties (16a, 16b) détachables l'une de l'autre par un mouvement (M1) de rotation relatif entre les deux parties ou par un mouvement axial relatif entre les deux parties et **en ce que** le collier (16b) forme une première partie de la gaine.

5. Seringue selon la revendication 4, **caractérisée en ce qu'**un jeu radial (J2) existe entre l'embout souple (14) et la deuxième partie (16a) de la gaine (16).

6. Seringue selon l'une des revendications 3 à 5, **caractérisée en ce que** l'embout (14) et la gaine (16) sont liés en translation parallèlement à l'axe longitudinal (X1).

7. Seringue selon la revendication 6, **caractérisée en ce que** la gaine (16) comprend deux épaulements annulaires opposés (168, 169) qui délimitent entre eux un volume (016) de réception d'un bourrelet annulaire (141) appartenant à l'embout souple.

8. Seringue selon l'une des revendications précédentes, **caractérisée en ce que** le collier (16b) comprend des pattes (166) d'accrochage sur le corps de seringue, ces pattes ne s'opposant pas à la rotation du collier autour de la partie d'extrémité (82) du corps de seringue (2) lorsque l'embout est retiré de l'aiguille.

9. Seringue selon l'une des revendications précédentes, **caractérisée en ce qu'**elle comprend des moyens (180d) de verrouillage du manchon (18) en position avancée en fin d'injection.

10. Seringue selon l'une des revendications précédentes, **caractérisée en ce que** la seringue est une seringue pré-remplie (1).

## Patentansprüche

1. Spritze (1) mit eingeklebter Nadel (10), umfassend:
- einen Spritzenkörper (2),
- einen Nadelschutz (12), der einen flexiblen Ansatz (14) aufweist,
- eine Außenhülse (18), die gemäß einer Längsachse (X1) zwischen einer vorgerückten Stellung, in der sie die Nadel bedeckt, und einer zurückgezogenen Stellung, in der sie die Nadel nicht bedeckt, beweglich ist und
- ein Kragenteil (16b), das mit einem radialen Spiel (J1) um ein Endteil (82) des Spritzenkörpers (2) herum montiert ist und das mindestens einen Vorsprung (160) aufweist, der in eine Öffnung (180) zur Führung der Hülse eingreift,
**dadurch gekennzeichnet, dass** ein Teilstück (143) des flexiblen Ansatzes (14) das radiale Spiel (J1) zwischen dem Endteil (82) des Spritzenkörpers und dem Kragenteil (16b) füllt, wenn der Ansatz (14) an der Spritze montiert ist, derart dass das Kragenteil hinsichtlich einer Drehung um das Endteil des Spritzenkörpers festgelegt ist, solange der Ansatz (14) an der Spritze montiert ist.

2. Spritze nach Anspruch 1, **dadurch gekennzeichnet, dass** das Teilstück (143) des flexiblen Ansatzes (14) radial zwischen dem Endteil (82) des Spritzenkörpers und dem Kragenteil (16b) komprimiert ist, wenn der Nadelschutz (12) an der Spritze montiert ist.

3. Spritze nach Anspruch 1 oder 2, **dadurch gekennzeichnet, dass** der Nadelschutz (12) eine starre Hülle (16) umfasst, die den flexiblen Ansatz (14) umhüllt.

4. Spritze nach Anspruch 3, **dadurch gekennzeichnet, dass** die Hülle als zwei Teile (16a, 16b) ausgebildet ist, die durch eine relative Drehbewegung (M1) zwischen den zwei Teilen oder durch eine relative axiale Bewegung zwischen den zwei Teilen lösbar sind, und dass das Kragenteil (16 b) einen ersten Bestandteil der Hülle bildet.

5. Spritze nach Anspruch 4, **dadurch gekennzeichnet, dass** ein radiales Spiel (J2) zwischen dem flexiblen Ansatz (14) und dem zweiten Teil (16a) der Hülle (16) vorhanden ist.

6. Spritze nach einem der Ansprüche 3-5, **dadurch gekennzeichnet, dass** der Ansatz (14) und die Hülle (16) translatorisch parallel zur Längsachse (X1) verbunden sind.

7. Spritze nach Anspruch 6, **dadurch gekennzeichnet, dass** die Hülle (16) zwei gegenüberliegende Ringschultern (168, 169) umfasst, die zwischen sich ein Volumen (O16) zur Aufnahme eines Ringswulstes (141), der zu dem flexiblen Ansatz gehört, begrenzen.

8. Spritze nach einem der vorhergehenden Ansprüche, **dadurch gekennzeichnet, dass** das Kragenteil (16b) Laschen (166) zur Befestigung an dem Spritzenkörper umfasst, wobei diese Laschen sich nicht der Drehung des Kragenteils um das Endteil (82) des Spritzenkörpers herum (2) entgegenstellen, wenn der Ansatz von der Nadel abgezogen wird.

9. Spritze nach einem der vorhergehenden Ansprüche, **dadurch gekennzeichnet, dass** sie Mittel (180d) zur Verriegelung der Hülse (18) in der vorgerückten Stellung am Ende der Injektion umfasst.

10. Spritze nach einem der vorhergehenden Ansprüche, **dadurch gekennzeichnet, dass** die Spritze eine vorgefüllte Spritze (1) ist.

## Claims

1. Syringe (1) with a bonded needle (10), comprising:
- a body (2) of a syringe,
- a needle guard (12), comprising a flexible tip (14),
- an outer sleeve (18), which is movable along a longitudinal axis (X1) between an advanced position where it covers the needle, and a retracted position where it does not cover the needle, and
- a collar (16b), which is mounted with a radial clearance (J1) around an end portion (82) of the syringe body (2), and which comprises at least one pin (160) engaged in a guide opening (180) of the sleeve,
**characterized in that** a part (143) of the flexible tip (14) fills the radial clearance (J1) between the end part (82) of the syringe body and the collar (16b) when the tip (14) is mounted on the syringe, so that the collar is immobilized in rotation about the end part of the syringe body as long as the tip (14) is mounted on the syringe.

2. Syringe according to claim 1, **characterized in that** the part (143) of the flexible tip (14) is compressed radially between the end part (82) of the syringe body and the collar (16b) when the needle guard (12) is mounted on the syringe.

3. Syringe according to claim 1 or 2, **characterized in that** the needle cover (12) comprises a rigid sleeve (16) enveloping the flexible tip (14).

4. Syringe according to claim 3, **characterized in that** the sleeve is in two parts (16a, 16b) detachable from one another by a movement (M1) of relative rotation between the two parts, or by relative axial movement between the two parts, and wherein the collar (16b) forms a first part of the sleeve.

5. Syringe according to claim 4, **characterized in that** a radial clearance (J2) exists between the flexible tip (14) and the second part (16a) of the sleeve (16).

6. Syringe according to one of the claims 3 to 5, **characterized in that** the tip (14) and the sleeve (16) are linked in translation parallel to the longitudinal axis (X1).

7. Syringe according to claim 6, **characterized in that** the sleeve (16) comprises two opposite annular shoulders (168, 169) which delimit between them a volume (O16) for receiving an annular bead (141) belonging to the soft tip.

8. Syringe according to one of the preceding claims, **characterized in that** the collar (16b) comprises tabs (166) for attachment to the syringe body, wherein these tabs do not oppose the rotation of the collar about the end part (82) of the syringe body (2) when the tip is removed from the needle.

9. Syringe according to one of the preceding claims, **characterized in that** it comprises means (180d) for locking the sleeve (18) in the advanced position at the end of injection.

10. Syringe according to one of the preceding claims, **characterized in that** the syringe is a pre-filled syringe (1).
